# EUROPEAN PATENT SPECIFICATION

(11) **EP 0 922 101 B1**
(45) Date of publication and mention of the grant of the patent: **17.12.2003**
(21) Application number: 97940036.3
(22) Date of filing: 28.07.1997
(51) Int. Cl.: C12N 15/12, C07K 14/475, A61K 38/18, C12N 1/21

(54) **EGF-LIKE FACTOR FROM CHROMAFFIN GRANULES AND GLIA CELL-DERIVED NEUROTROPHIC FACTOR WITH SURVIVAL-PROMOTING ACTIVITY ON DAERGIC NEURONS**
EGF-ÄHNLICHER FAKTOR VON CHROMAFFIN GRANULA UND AUS GLIAZELLEN ABSTMMENDER FAKTOR MIT ÜBERLEBENSFÖRDERNDER AKTIVITÄT AUF DOPAMINERGISCHEN NEURONEN
FACTEUR SEMBLABLE A EGF DERIVE DE GRANULES DE CHROMAFFINE ET FACTEUR NEUTROTROPHIQUE DERIVE DE CELLULES GLIA PRESENTANT UNE ACTIVITE FAVORISANT LA SURVIE DE NEURONES DAERGIQUES

(30) Priority: 29.07.1996 EP 96112218; 09.08.1996 US 24143 P
(43) Date of publication of application: 16.06.1999
(73) Proprietor: BIOPHARM Gesellschaft zur biotechnologischen Entwicklung und zum Vertrieb von Pharmaka mbH, 69115 Heidelberg (DE)
(72) Inventor: UNSICKER, Klaus, D-69118 Heidelberg (DE); PAULISTA, Michael, D-69181 Leimen (DE); POHL, Jens, D-76707 Hambrücken (DE); BECHTOLD, Rolf, D-69126 Heidelberg (DE)
(74) Representative: Müller-Boré & Partner Patentanwälte
(86) International application number: EP9704087
(87) International publication number: WO98004688

(56) References cited:
- EP-A- 0 200 341
- WO-A-93/06116
- KRIEGLSTEIN, K. ET AL.: "Bovine chromaffin cells release a transforming growth factor-beta-like molecule contained within chromaffin granules" JOURNAL OF NEUROCHEMISTRY, vol. 65, no. 3, September 1995, pages 1423-1426, XP002050426
- LACHMUND, A. ET AL.: "Trophic factors from chromaffin granules promote survival of peripheral and central nervous system neurons" NEUROSCIENCE, vol. 62, no. 2, September 1994, pages 361-370, XP002050427 cited in the application
- UNSICKER, K. ET AL.: "Growth factor function in the development and maintainance of midbrain dopaminergic neurons: concepts, facts and prospects of TGF-beta" CIBA FOUNDATION SYMPOSIUM, vol. 196, 1996, CHICHESTER, pages 70-84, XP002050429 cited in the application
- DATABASE BIOSIS BIOSCIENCES INFORMATION SERVICE, PHILADELPHIA, PA, US AN 97:471729, 1997 HENHEIK, P. ET AL.: "Co-release of GDNF and TGF-beta accounts for the neurotrophic activity of chromaffin granule protein" XP002050595 & SOCIETY FOR NEUROSCEINCE ABSTRACTS, vol. 23, no. 1-2, 1997, page 889
- KRIEGLSTEIN, K. ET AL.: "Protein from chromaffin granules promotes survival of mesencephalic dopaminergic neurons by an EGF-receptor mediated mechanism" JOURNAL OF NEUROSCIENCE RESEARCH, vol. 48, 1 April 1997, pages 18-30, XP002050428

## Description

The present invention relates to chromaffin granule-derived EGF-like proteins that are involved in promoting survival of DAergic neurons, and to processes for their preparation.

Grafts of adrenal medullary tissue and chromaffin cells to the striatum of patients with Morbus Parkinson (PD) and animal models of the disease have pronounced therapeutic effects leading to the restoration of transmitters and motor functions (for reviews, see Freed, 1993; Fisher and Gage, 1993). A common explanation for these effects has been that grafted chromaffin cells may compensate, by secreting dopamine (DA), for the substantial lack of this nigrostriatal transmitter in PD (Ehringer and Hornykiewicz, 1960). However, amounts of DA synthesized and released by chromaffin cells are small (approx. 1 % of total catecholamines in adult rat; Coulter et al., 1988), and increases of striatal DA are even very modest immediately adjacent to a chromaffin cell graft (Becker and Freed, 1988). It has therefore also been argued that the release of chemical mediators other than DA may be a cause for the beneficial effects of chromaffin cell grafts to the Parkinsonian brain. Substantial sprouting of remaining axons within the lesioned striatum (Bohn et al., 1987; Kordower et al., 1991) has raised speculations that neurotrophic molecules or cytokines eliciting synthesis and relase of neurotrophic molecules may underly the curative effects of chromaffin cell grafts. This notion receives support from increasing evidence that chromaffin cells synthesize, store, and release a large number of neurotrophically active growth factors and neuropeptides (Lachmund et al., 1994; Unsicker and Stögbauer, 1992; see Unsicker, 1993, and Unsicker and Krieglstein, 1996, for reviews). Stimulation of granule release from chromaffin cells with the cholinergic agonist carbachol was shown to increase the output of neurotrophic factor activity for several neuron populations of the peripheral and central nervous system (Lachmund et al., 1994). The depolarization-induced secretion of neurotrophic activity was parallelled by increased release of chromo-granin A and catecholamines suggesting a co-release from chromaffin granules.

For a long time, basic fibroblast growth factor (FGF-2) has been suspected to be responsible for at least some of the curative actions of chromaffin cells grafted to the lesioned nigrostriatal system, since FGF-2 can fully mimic the trophic effects of the grafts (Otto and Unsicker, 1990, 1993a). However, FGF-2 is not located in chromaffin granules and is not released via the regulated or constitutive pathways of secretion (Bieger et al., 1995; Stachowiak et al., 1994).

Accordingly, the technical problem underlying the present invention is to provide new compounds capable of promoting survival of (mesencephalic) DAergic neurons, which can be used for the treatment of peripheral and/or CNS-disorders in man.

These compounds can be the EGF-like activity from chromaffin granules as well as functional parts thereof or chemical compounds serving the same function which are small enough to cross the blood-brain barrier to manage that function.

The solution to the above technical problem is achieved by the embodiments characterized in the claims.

In particular, the present invention relates to a chromaffin granule-derived protein mediating selectively survival promoting activity on DAergic neurons. The term "chromaffin granule-derived protein" refers to single, defined proteins that, when applied singly or in combinations exert trophic, survival and differentiation promoting effects on DAergic neurons. The expression "selective survival promoting acitivity on DAergic neurons" refers to a proteinaceous activity that may confer, by itself or in combination with other factors present in chromaffin granules, survival and differentiation upon DAergic neurons within the nanomolar range or below.

The chromaffin granule-derived protein of the present invention is an epidermal growth factor receptor (EGFR)-ligand; i.e. the chromaffin granule-derived protein exhibits effects within a nanomolar range of concentrations.

The chromaffin granule-derived protein of the present invention is capable of promoting astroglial cell maturation. The expression "capable of astroglial cell maturation" means that within a culture system of embryonic mesencephalic cells, the protein increases the number of astroglial cells visualized by expression of proteins that are specific for this cell type.

In a preferred embodiment of the present invention, the chromaffin granule-derived protein is capable of inducing proliferation of non-DAergic cells, which are presumed to be glial progenitor cells. The expression "capable of proliferation of non-DAergic cells" means that expansion of number of presumed astroglial progenitor cells is the effect initially caused by the "chromaffin granule-derived protein". This initial effect is supposed to be the prerequisite for the promotion of survival by a factor secreted by the expanded number of astroglial cells.

A further subject of the present invention relates to a process for the preparation of the above defined chromaffin granule-derived protein, comprising the steps of isolating chromaffin granules from chromaffin cells and extracting the aqueous-soluble protein content containing the chromaffin granule-derived protein, from the chromaffin granules in a buffer solution. The expression "isolating chromaffin granules from chromaffin cells" comprises subcellular fractionation of chromaffin cell organelles by density gradient centrifugation at 1.7M sucrose. The expression "extracting the aqueous-soluble protein content containing the chromaffin granule-derived protein" comprises lysis of the organelles obtained as a pellet of the 1.7M sucrose centrifugation in a 10 mM phosphate buffer at pH 7.0 following twenty minutes freezing at -80°C.

A further subject of the present invention relates to a pharmaceutical composition comprising the above defined chromaffin granule-derived protein, optionally in association with a pharmaceutically acceptable carrier and/or diluent. The pharmaceutical composition may be used for the treatment of peripheral and/or CNS-disorders in man such a Parkinson's disease, Alzheimer's disease or other dementias, other neurodegenerative disorders of the central nervous system and peripheral neuropathies including diabetes, cisplatinium neuropathy or other genetic or acquired peripheral nerve diseases.

A further subject of the present invention comprises the application of the above factor derived from chromaffin granules in conjunction with previously found neurotrophic molecules, such as neurotrophins, members of the TGF-β, fibroblast growth factor, and neuropoietic cytokine superfamilies.

The Figures show:

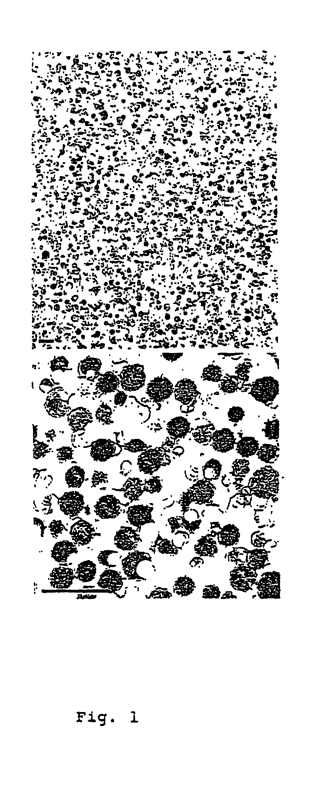

*Figure 1*. Electron micrographs of representative preparations of bovine chromaffin granules. Bar = 1 µm.

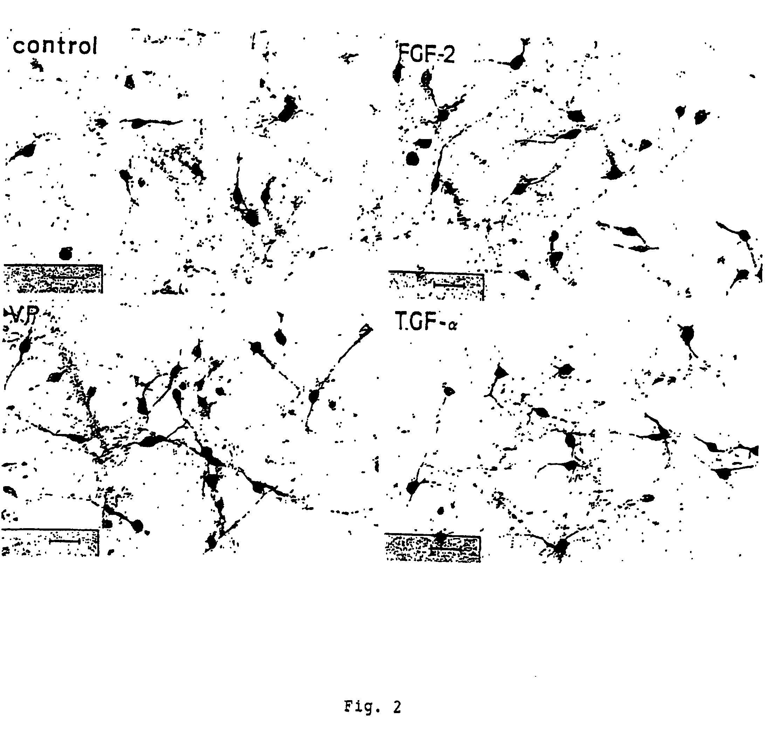

*Figure 2*. Photomicrographs of dissociated cultures of rat E14 ventral mesencephalic cells after 8 days in culture stained with monoclonal antibodies against TH. Cultures were control cultures and cultures treated with chromaffin granule-derived protein (VP) at a dilution of 1 :20, FGF-2 (10 ng/ml), or TGF-α (20 ng/ml). Bar = 25 µm.

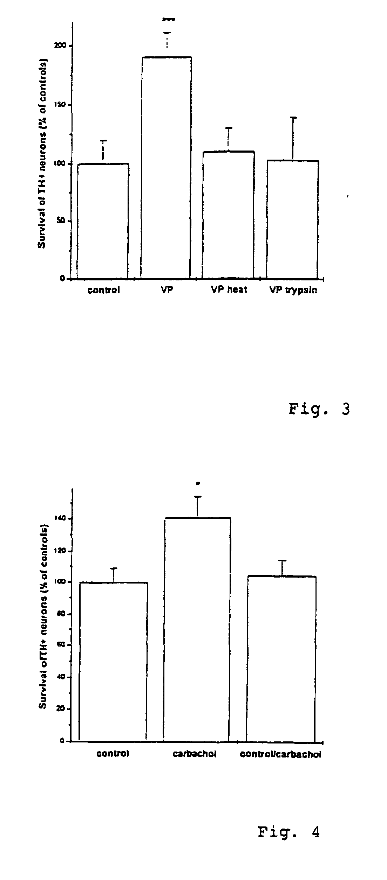

*Figure 3*. The survival promoting effect of chromaffin granule-derived activity is due to a protein. Cultures were treated for 7 days, starting 24 after seeding, with chromaffin granule-derived protein (VP) at a dilution of 1:20. Sister cultures were treated with identical amounts of heat-inactivated chromaffin granule-derived protein (VP heat), or trypsin-digested chromaffin granule-derived protein (VP trypsin). Each bar represents the mean number of TH-positive cells counted in triplicate cultures +/- SEM from two experiments. Intergroup differences were considered significant at ***P<0.001.
*Figure 4*. Upon cholinergic stimulation bovine chromaffin cells release the survival promoting activity for DAergic neurons into the medium. Cultures established from rat E 14 ventral mesencephalic cells were treated for 7 days, starting 24 after seeding, with conditioned medium from carbachol-stimulated chromaffin cells, or conditioned medium from unstimulated chromaffin cells without carbachol (control), or carbachol (10⁻⁵ M) containing culture medium. Each bar represents the mean number of TH-positive cells counted in quadruplicate cultures +/- SEM from two experiments. *P<0.05

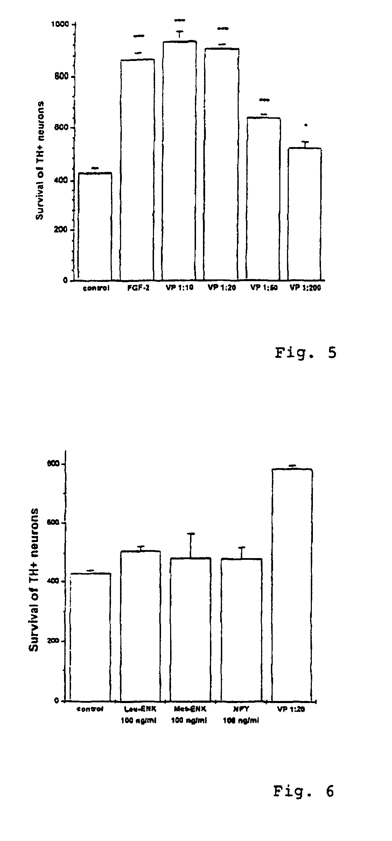

*Figure 5*. Dose-response curve of the chromaffin granule-derived activity (VP) on the survival of TH-immunoreactive neurons of mesencephalic cultures (E14) at the end of the eight-day culture period. Cultures were treated with VP at dilutions of 1:10, 1:20, 1:50 and 1:200, or with FGF-2 (10 ng/ml). Results are mean +/- SEM of triplicate determinations, of two replicate experiments. *P<0.05, ***P<0.001.

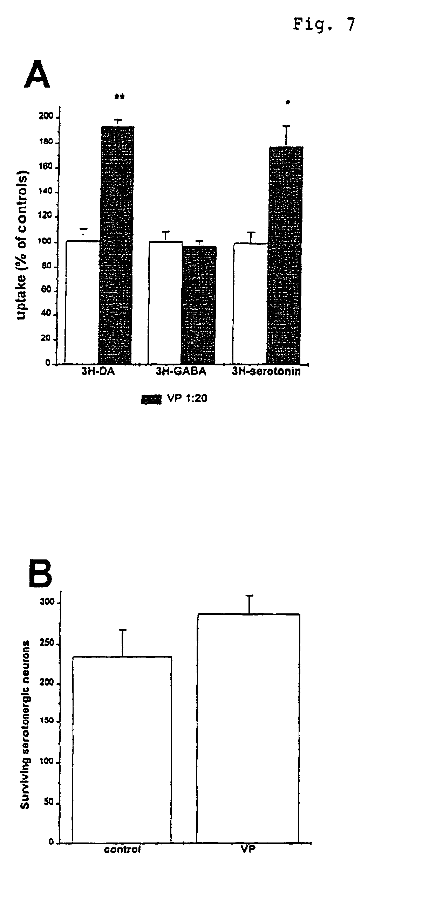

*Figure 6*. The survival promoting effect of chromaffin granule-derived activity is not due to enkephalin or neuropeptide Y. Cultures were treated for 7 days, starting 24 after seeding, with Leu-enkephalin (Leu-ENK), Met-enkephalin (Met-ENK), neuropeptide Y (NPY) at 100 ng/ml or with chromaffin granule-derived protein (VP) at a dilution of 1:20. Each bar represents the mean number of TH-positive cells counted in triplicate cultures +/- SEM from two experiments.
*Figure 7*. ³H-DA, ³H-GABA-, and ³H-serotonin-uptake (A) and the survival of serotonin-immunoreactive neurons (B) is shown for mesencephalic cultures (E14) at the end of the eight-day culture period. The effect of chromaffin granule-derived protein (VP) at a dilution of 1:20 is documented. Controls of uptake studies were set to 100 percent. Results are mean +/- S.E.M. of triplicate determinations, of two experiments. *p< 0.05, **P<0.01.

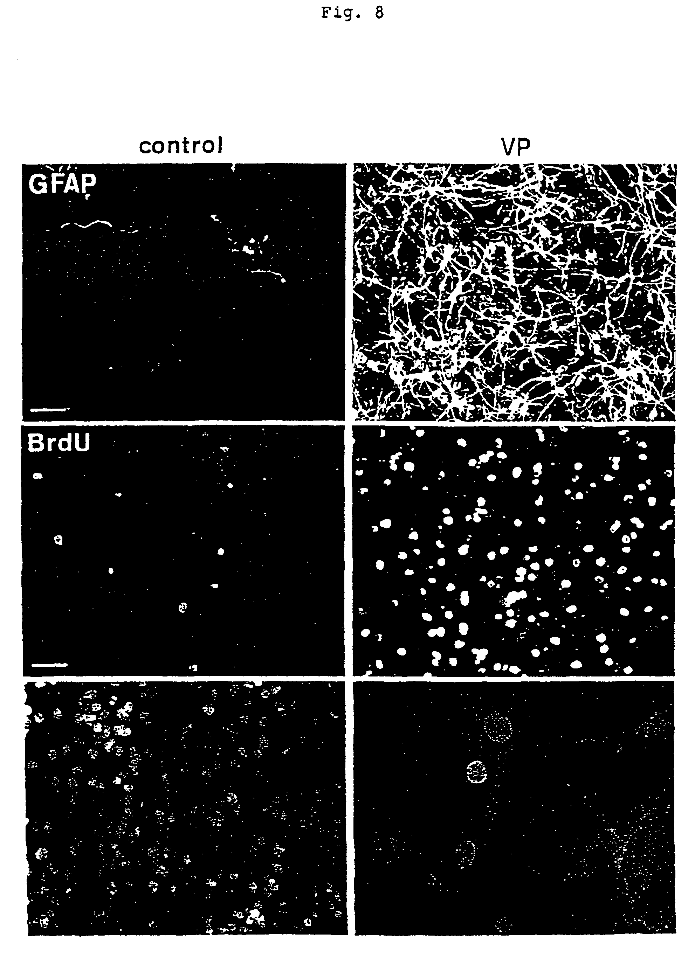

*Figure 8*. Photomicrographs of dissociated cultures of rat E14 ventral midbrain floor after 8 days in culture. Upper panels show staining with monoclonal antibody against GFAP; middle panels show the visualization of BrdU incorporation using monoclonal antibodies against BrdU. Bottom panels show nuclear staining using propidium iodide (left) and (right) double-labeling for TH (red) and BrdU (green). Cultures shown at the left hand side represent control cultures; panels on the right hand side are VP-treated cultures at a dilution of 1:10. Bar = 25 µm.

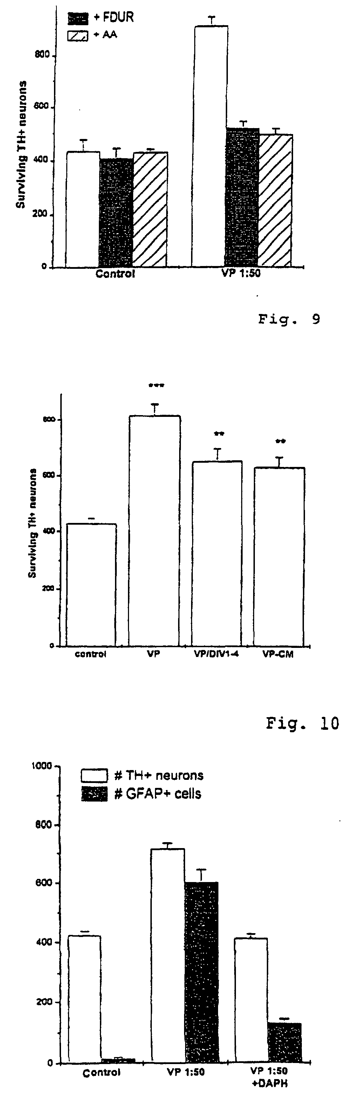

*Figure 9*. Numbers of surviving TH-immunoreactive neurons of mesencephalic cultures (E14) at the end of the eight-day culture period. The antimitotic agent FOUR and the gliotoxin AA were used at a concentration of 30 µM each in control cultures, or in cultures treated with VP at a dilution of 1:50. Results are mean +/- S.E.M. of triplicate determinations, of two experiments.
*Figure 10*. Numbers of surviving TH-immunoreactive neurons of mesencephalic cultures (E14) at the end of the eight-day culture period. Cultures were treated either with chromaffin granule-derived protein at a dilution of 1:20 from DIV1-DIV8 (VP), or from DIV1-DIV4 followed by treatment with culture medium only (VP/DIV1-4), or treatment with VP-conditioned medium (VP-CM). Results are mean +/- S.E.M. of triplicate determinations, of two experiments. **P<0.01, ***P<0.001.

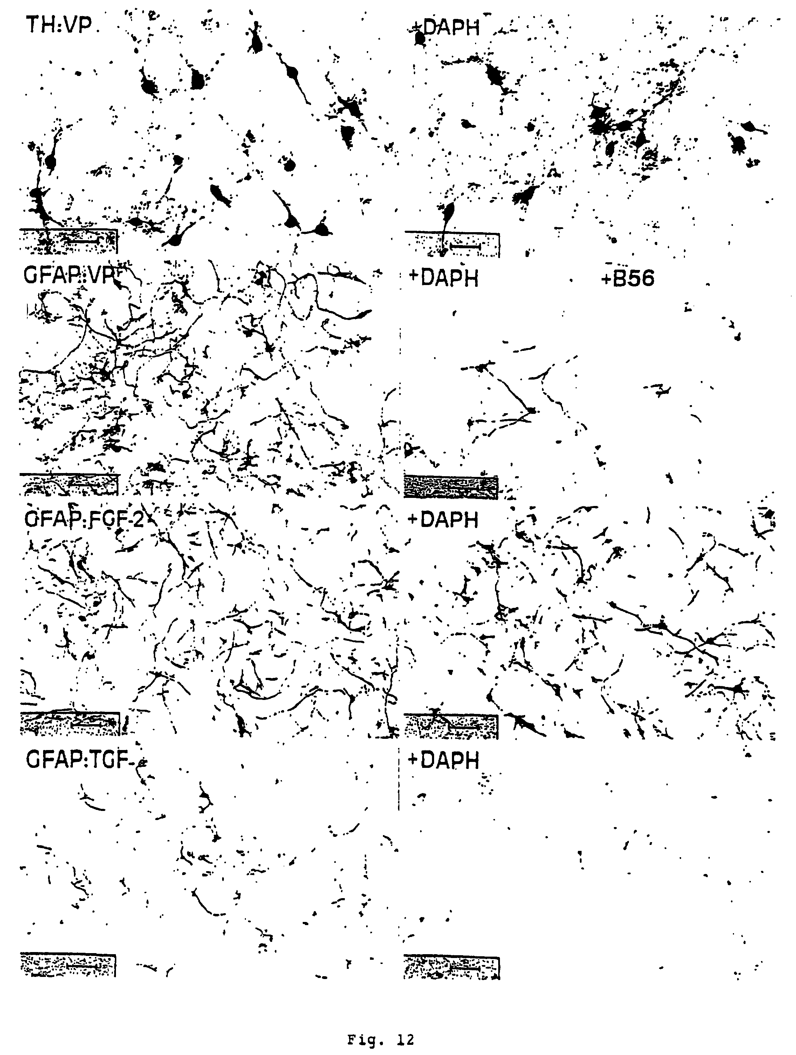

*Figure 11*. The survival promoting effect of chromaffin granule-derived protein (VP) on DAergic neurons can be abolished by using DAPH, an inhibitor of the EGFR signaling pathway. Cultures derived from E14 rat ventral mesencephalon were treated with VP at a dilution of 1:50 in the absence or presence, respectively, of 10 µM of DAPH. Untreated cultures served as controls. At DIV 6 cultures were fixed and processed for GFAP or TH immunoreactivity. Numbers of TH positive cells (open bars) are given as mean + /- SEM of triplicate determinations of two replicate experiments, and numbers of GFAP positive cells are given as mean of two determinations of two replicate experiments.
*Figure 12*. The appearence of GFAP positive astroglial cells induced by chromaffin granule-derived protein, or TGF-α, but not FGF-2 can be inhibited by DAPH, an EGFR signaling pathway inhibitor. Cultures derived from E14 rat ventral mesencephalon were treated with VP at a dilution of 1:50, FGF-2 at 10 ng/ml or TGF-α at 20 ng/ml in the absence (left panels) or presence, respectively, of 10 µM of DAPH or Tyrphostin B56 (right panels). At DIV 6 cultures were fixed and processed for TH (upper panel) or GFAP immunoreactivity, followed by DAB visualization. Bar = 25µm

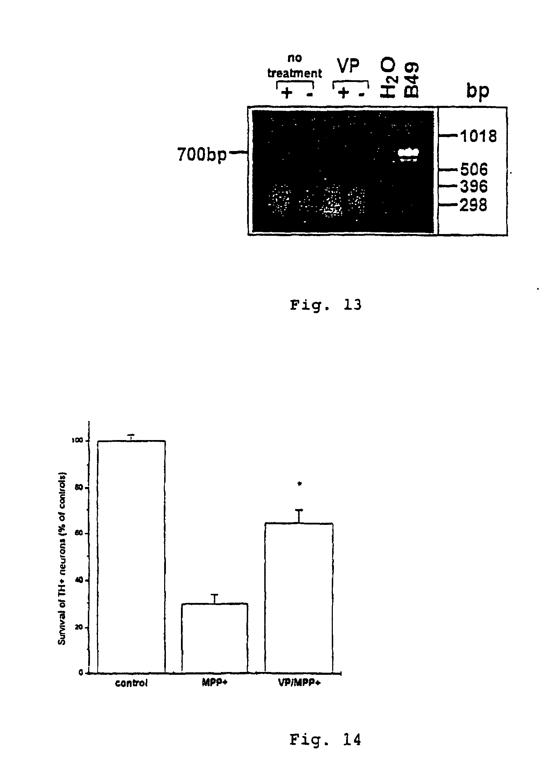

*Figure 13*. Expression of GDNF in mesencephalic cell cultures (DIV8) detected by RT-PCR. - lanes represent controls, in which total RNA was not transcribed, + lanes represent cDNA. A positive signal for GDNF (700 bp) is obtained from B49 glioma cells, but not from VP-treated or untreated mesencephalic cell cultures.
*Figure 14*. The chromaffin granule-derived protein protects against MPP+ toxicity. Percentage of surviving TH + neurons after treatment with MPP+ (1µM), or MPP+ in combination with VP 1:20 are shown. Controls were set as 100%. Results are mean +/- SEM of triplicate determinations, of two replicate experiments. *P<0.05

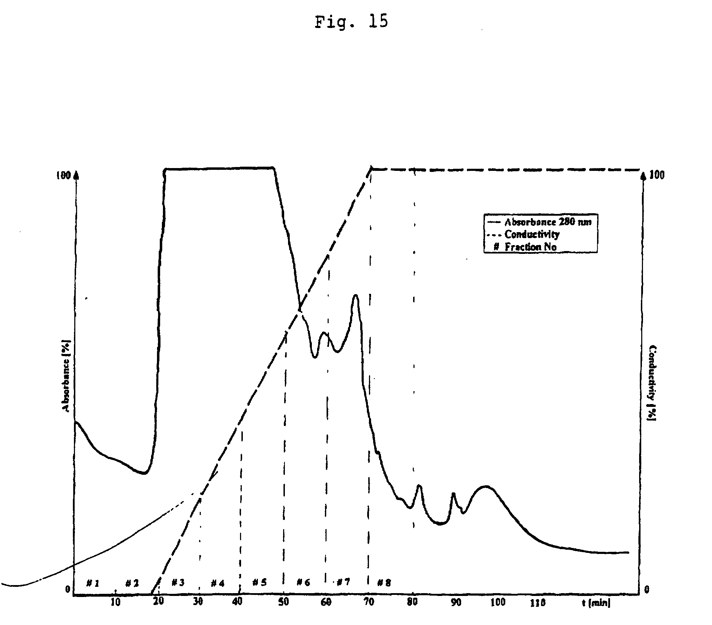

*Figure 15*. Chromatogram of the purification of the chromaffin granule protein on Heparin Sepharose.

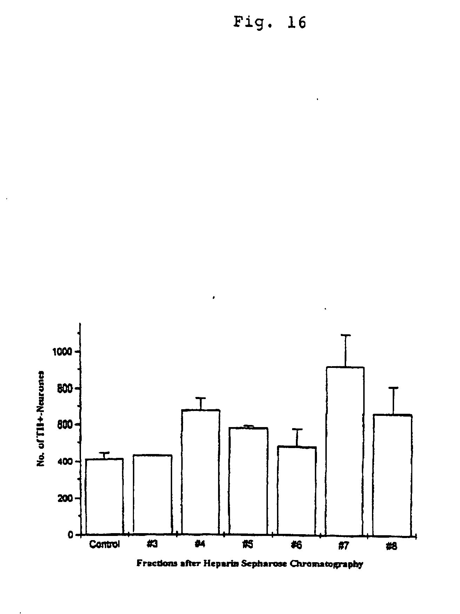

*Figure 16*. Numbers of surviving TH-immunoreactive neurons of mesencephalic cultures (E14) at the end of the eight-day culture period, using the protein fractions after Heparin Sepharose chromatography.

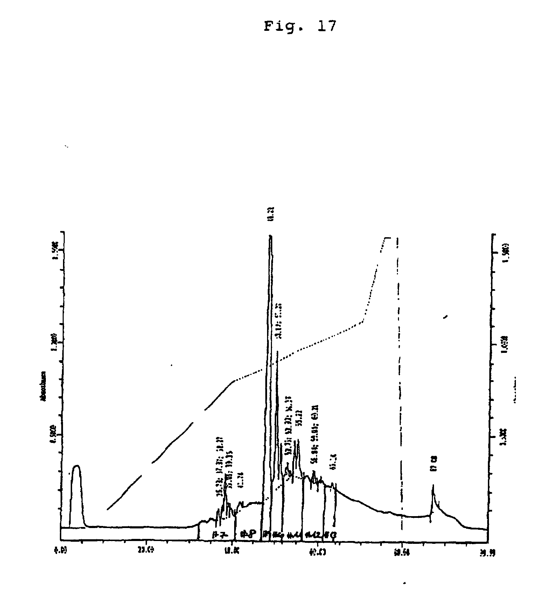

*Figure 17*. Chromatogram of the purification of the chromaffin granule protein using RP-HPLC.

The following Examples illustrate the invention:

*Culture medium, growth factors and chemicals*. The serum-free medium used consisted of Dulbecco's modified Eagle's medium and Ham's F-12 (DMEM/F-12, BioWhittaker), including the N1 supplements (Bottenstein et al., 1980) apotrans-ferrin, insulin, L-thyroxine, sodium selenite and progesterone (Sigma, Deideshofen, Germany), and 2.5 mM glutamine (GIBCO, Eggenstein, Germany), 2.5 mg/ml bovine serum albumine (Sigma), 100 U/ml penicillin, 100 µg/ml streptomycin, and 0.25 µg/ml fungizone (BioWhittaker, Heidelberg, Germany). Calcium-magnesium- free Hank's balanced salt solution (CMF), horse serum, trypsin, 5-fluorodeoxyuridine (FDUR), and α-aminoadipic acid (AA) were from Sigma, DNAse was from Boehringer. 4,5-Dianilinophthalimide (DAPH) and Tyrphostin B56 (Calbiochem, Bad Soden, Germany) were dissolved in DMSO to give a final concentration of 10 mM, protected from light and stored at 4°C until use. FDUR and AA were dissolved in PBS to give a final concentration of 1mM and stored in aliquots at -20°C.

Spectrapor dialysis tubing (Roth, Karlsruhe, Germany) with a cutoff MW 3,500 was used. Growth factors: rh FGF-2, rh TGF-α, rh EGF (IC Chemikalien, Ismaningen, Germany). Lyophylized growth factors were resuspended in DMEM containing 0.25% BSA and penicillin, streptomycin, and fungizone in the given concentration, to give a final concentration of 1µg/ml. Aliquots of 100 µl were kept at -70°C until used.

*Isolation of soluble chromaffin granule content*. The isolation procedures follows essentially the protocol of Winkler and Smith (1975). Briefly, approx. 60 bovine adrenals obtained from the slaughter-house, Mannheim, Germany, were dissected, medullae pooled in 0.3 M sucrose, 10 mM phosphate buffer, pH 7.0, homogenized, centrifuged for 15 min at 380 g, followed by centrifugation of the supernatant at 8,720 g for 20 min. The pellet is known to contain all organelles, which can then be further factionated by sucrose gradients. In order to purify large dense core vesicles, the chromaffin granules, the resuspended pellet (0.3 M sucrose, 10 mM phosphate, pH 7.0) was loaded on a 1.7 M sucrose cushion. The chromaffin granules were obtained as a sediment after centrifugation at 100,000 g for 90 min. The sediment was resuspended in 10 mM phosphate pH 7.0, frozen in liquid nitrogen and subsequently thawed, in order to lyze the vesicles and to extract the soluble protein content. Membrane fragments were collected by centrifugation at 100,000g for 30 min. The supernatent containing the soluble protein mixture from chromaffin granule was dialysed (cutoff 3,500 MW) over night against several batches of 100-fold excess of 10 mM phosphate buffer pH 7.0 to seperate catecholamines and other low molecular weight components from proteins. To quantify protein concentrations the Bradford (Bio-Rad) protein assay was employed using bovine gamma globulin as a standard (Bio-Rad). The protein solution was diluted to give a final protein concentration of 20 mg/ml. This protein solution was then sterile filtered (0.22 µM) and stored in aliquots at -70°C. For chromatography on Heparin-Sepharose (Econo-Pac Heparin Cartridge, 5 ml BioRad) the purified granules were loaded in 100 mM NaCl, 10 mM Tris, 0.1 % Chaps at pH 7.3. After washing with the same buffer, the protein was eluted using a gradient from 100 mM NaCl to 1 M NaCl in 10 mM Tris, 0.1 % Chaps at pH 7.3. The fractions collected were desalted by reversed phased chromatography (RP-HPLC) on Aquapore RP-300 columns, 7 micron, Applied Biosystems, using running buffer A (0.1 % TFA) and running buffer B (0.1 % TFA/90 % Acetonitril). For the determination of the biological activity, the lyophilized samples were resuspended in 20 µl of 50 % Acetonitril and added to the medium at 5 µl per 2 ml Medium at each medium change. Heat inactivation was achieved by heating a protein sample three times to 90°C for 30 sec. An aliquot was digested with 0.1% trypsin at 37°C for one hour. Digestion was terminated by adding 100 µg disolved in 50 µl PBS of soybean trypsin inhibitor.

*Electron microscopy*. Isolated chromaffin granules were pelleted and fixed with 1.5% formaldehyde and 1.5% glutaraldehyde in phosphate buffer (pH 7.4) followed by 1% osmiumtetroxide in 1.5% potassiumhexacyanoferrat-II. After blockstaining in 1 % aqueous uranly acetate and dehydration in ethanol, the pelets were embedded in Epon, sectioned on a LKB ultratome III and viewed in a Zeiss EM10 electron microscope.

*Tissue culture*. Mesencephalic cell cultures were essentially established as described by Krieglstein and Unsicker (1994). In brief, the ventral midbrain floor was dissected from embryonic day (E) 14 Wistar rat fetuses of two litters (20-25 embryos) and collected in CMF. Tissue pieces were enzymatically dissociated using 0.25% trypsin (BioWhittaker) in CMF for 15 min at 37°C. After addition of an equal volume of ice-cold horse serum and 1mg DNAse, Cells were triturated with fire-polished and siliconized pasteur pipettes and subsequently washed with DMEM/F12. The single cell suspension (100µl) was seeded on polyornithine (0.1 mg/ml in 15mM borate buffer, pH 8.4, Sigma)-laminin (5µg/ml; Sigma) coated glass cover slips at a density of 200.000 cells/cm². Coverslips were incubated in a humified 5% CO₂/95% air atmosphere to allow cells to attach. After two hours coverslips were transferred to 24-well plates (Falcon) containing 750 µl medium. On the following day, and subsequently every three days, 500 µl of the medium was replaced and neurotrophic factors were added at the same time at the given concentrations.

Bovine chromaffin cells were isolated by collagenase perfusion and digestion as previously described and enriched to > 95% purity employing Percoll gradient centrifugation (Unsicker et al., 1980; Bieger et al., 1995). Chromaffin cells were seeded at 200,000 cells/cm² on plastic culture flasks (Falcon; 5x10⁶ cells per 25cm²) and maintained in 5 ml of DMEM with N1 supplements for 40h. After washing of cells with prewarmed medium cells were exposed to 2ml DMEM/N1 containing the cholinergic agonist carbachol (10⁻⁵ M) for 15 min, while control cultures were treated identically, but without secretagogue (cf. Lachmund et al., 1994). Conditioned medium from stimulated and unstimulated cells was stored in aliquots at -80°C to avoid repeated freezing and thawing and applied at 1:4 dilution to cultures of mesencephalic DAergic neurons.

*Immunocytochemistry*. To identify DAergic or serotinergic neurons cells were visualized using antibodies aginst tyrosine hydroxylase (TH), or serotonin, respectively. Cells were fixed with 4% paraformaldehyde buffered in phosphate buffered saline (PBS) for 10 min at room temperature, permeabilized with acetone at -20°C for 10 min and washed with (PBS). After blocking with 1% H₂O₂ in PBS, followed by 1 % horse serum, coverslips were stained with a monoclonal antibody to rat TH (1:200; Boehringer Mannheim; diluted in 1 % horse serum) or with an antibody against serotonin (1:50; DAKO) for 1 h at 37°C. Specific staining was vizualized using the anti-mouse Vectastain ABC kit in combination with DAB (Camon, Germany). For glial fibrillary acidic protein (GFAP) immunocytochemistry, cells were fixed and permeabilized using aceton at -20°C for 20 min, then washed with PBS and incubated with a monoclonal antibody against GFAP (1:100; Sigma) for 1 h at 37°C. As a secondary antibody TRITC anti-mouse-IgG was used. To monitor cell proliferation bromodesoxyuridine (BrdU) was added to the culture, 24 hours prior fixation, at a final concentration of 10 µM. Cells were washed twice with PBS and fixed with 70% ethanol buffered with 50 mM glycine, pH 2.0, for 20 min at -20°C. Incorporated BrdU was identified using anti-BrdU detection Kit I (Boehringer, Mannheim). BrdU/TH double detection was achieved by first applying the protocol for BrdU followed after another five washes with PBS and by the procedure for TH staining using TRITC anti-mouse-IgG as a secondary antibody. Nuclei were stained with propidium iodide (20 s, 0.1µg/ml). Coverslips were mounted using Aquatex (Merck, Darmstadt).

*Transmitter uptake studies*. High affinity uptake of ³H-DA, ³H-GABA and ³H-serotonin (Amersham) were determined according to a modified method described by Alexi and Hefti (1993). Cells were washed three times with the incubation solution (5 mM glucose and 1 mM ascorbic acid in PBS, pH 7.4), preincubated for 15 min at 37°C with 0.5 ml of incubation solution, before adding 50 nM ³H-GABA or ³H-serotonin for a further 15 min period. Blanks were obtained by incubating the cells at 4°C. Uptake was stopped by removing the incubation mixture, followed by three rapid washes with ice-cold PBS. Then 300 µl of distilled water were added, cultures frozen for 2h at -80°C, thawn, and cells were scraped twice with an additional volume of 200 µl of distilled water. The extracted radioactivity was measured by liquid scintillation spectrometry after addition of 10 ml scintillation cocktail to each vial.

*Treatment of cultures with MPP*^{*+*}*, 5-fluorodeoxyuridine, and α-aminoadipic acid*. N-methyl-4-phenylpiridinium ion (MPP⁺; RBI) was added at day 4 to mesencephalic cell cultures at a final concentration of 1 µM for a period of 48h, The toxin was then replaced by fresh culture medium for a final 48h period. Treatment with chromaffin granule protein started at 24h prior to the addition of toxin and lasted until the end of the experiment (day 8).

5-Fluorodeoxyuridine (FDUR, Sigma), or α-aminoadipic acid (AA, Sigma) were added to cultures at a final concentrations of 30 µM in conjunction with each change of medium (O'Malley et al., 1994).

*Evaluation of cell numbers*. Survival of DAergic neurons was evaluated by counting all TH-positive neurons in one diagonal strips of the coverslip using 100-fold magnification. This area corresponded to 12% of the total area. Quantification was done in triplicate and in at least two independent experiments. The number of GFAP-positive cells were assessed likewise.

*RT-PCR*. At DIV 8 of chromaffin granule protein-treated or non-treated mesencephalic cell cultures total RNA was isolated by acid guanidinium thiocyanate/phenol/chloroform extraction from 10 wells and pooled (corresponding to 2x10⁶ cells). cDNA was synthesized in a final volume of 20 µl with the following components: 2.5 µg of total RNA, 50 mM Tris-HCl (8.3), 75 mM KCI, 3 mM MgCl2, 10 mM dithiothreitol, 5 mM dNTPs, 25 mM oligo(dT) primer (Gibco), 20 units of RNase inhibitor (Boehringer) and 200 units SuperScript RNase H⁻ reverse transcriptase (Gibco). The mixture was incubated at 37°C for 60 min. PCR was performed in a total volume of 25 µl containing cDNA (made from 625 ng of total RNA), 10 mM Tris-HCl (pH 8.8), 10 mMKCl, 2.5 mM MgCl₂, 0.002% Tween 20, 0.2 mM dNTPs, 1 µM 5'primers, 1 µM 3'primers, and 1.5 units UITma DNA polymerase (Perkin Elmer) using the hot start technique according to manufacturers instructions, in a Perkin Elmer GeneAmp PCR system 9600. The amplification steps involved denaturation at 94°C for 1 min, annealing for 2 min at 60°C with GDNF primers, and extension at 72°C for 3 min. Samples (5 µl) of the PCR mixtures were analyzed by electrophoresis in 2 % agarose gels in the presence of ethidium bromide. The GDNF primers were used as described by Schaar et al. (1993), and the expected size of the PCR product was 700 base pairs. RNA isolated from B49 cells, the glial cell line from which GDNF was originally isolated (Lin et al., 1993), was used as a positive control for GDNF amplification.

*Statistics*. The data were analysed by a one-way ANOVA, and the significance of intergroup differences was determined by applying Student's t-test. Differences were considered significant at *P<0.05, **P<0.01, ***P<0.001.

### Purification of chromaffin granules and assessment of purity

Bovine chromaffin granules were prepared essentially following a protocol by Winkler and Smith (1975). The pellet obtained representing the granule fraction was analysed by electron microscopy in order to detect contaminations with other organelles. A representative micrograph of a chromaffin granule preparation is shown in Fig. 1. Electron microscopic analysis revealed that these preparations are essentially composed of large dense core granules, identified by their density and size of approximately 280 nm. Granules were ruptured using a low salt buffer, in order to elute only soluble proteins, which are not membrane attached. Extensive dialysis was used to dilute out catecholamines and other small components, resulting in a protein mixture carefully analyzed by Winkler et al. (1986). The prominent protein constituents of chromaffin granules are chromogranins A and B, enkephalins, neuropeptide Y, and dopamine-β-hydroxylase (not shown).

### Soluble chromaffin granule protein promotes in vitro survival of DAergic neurons

To evaluate possible survival-promoting effects of chromaffin granule protein on DAergic neurons, cultures were treated with this mixture at a dilution of 1:20, starting treatment at day 1 in vitro (DIV 1). Fig. 2 shows that protein from chromaffin granules clearly maintains more TH-positive neurons as compared to control cultures after eight days in culture. This effect was comparable to that seen by treating the cultures with a saturating concentration of FGF-2 (10 ng/ml) or TGF-α (20 ng/ml).

In order to verify that this survival promoting effect is in fact due to a proteinaceous component, the protein sample was incubated with heat and trypsin. Either of the two procedures abolished the survival promoting activity (Fig. 3), indicating that the neurotrophic activity isolated from chromaffin granules is a protein.

### DAergic activity is released from chromaffin cells by stimulation with carbachol

To provide evidence that the DAergic activity was stored in *and* released from chromaffin granules medium conditioned by bovine chromaffin cells was applied to cultures of E14 mesencephalic DAergic neurons. Conditioned medium from cells stimulated with the cholinergic agonist carbachol (10⁻⁵M) increased survival of TH-positive neurons to 141.9 +/- 13.5% (n=4; Fig, 4) of control values (conditioned medium of unstimulated cells) at DIV 8. Culture medium containing carbachol was uneffective (Fig. 4). Thus, chromaffin granules store and release a neurotrophic activity supporting midbrain DAergic neurons.

### Dose dependence of chromaffin granule protein-mediated survival of DAergic neurons

Quantification of the neurotrophic effect of chromaffin granule protein showed highest survival promoting effects at a 1:20 dilution, and could not be further increased by using a dilution of 1:10. The IC₅₀ was reached at a dilution of 1:50 (Fig. 5).

### The trophic effect of chromaffin granule protein is not due to neuroactive neuropeptides contained in chromaffin granules

In order to investigate whether prominent components of chromaffin granules that are known to have some trophic activities (Unsicker and Stögbauer, 1992), can mimic the observed effects, neuropeptide Y, Leu- and Met-enkephalin were analysed for their capacity to promote survival of midbrain DAergic neurons. However, none of these neuropeptides had any survival promoting activity using concentrations up to 100 ng/ml (Fig. 6).

### The trophic effect of chromaffin granule protein is selective for DAergic neurons

DAergic neurons account for 5-10 % of the neuron population in the cell culture system employed as well as in the mesencephalon in vivo. Non-DAergic neurons are mainly GABAergic, but there are also few serotonergic neurons. To analyse the selectivity of the survival promoting effect of chromaffin granule protein, its effect on the uptake of DA, GABA and serotonin was investigated, following an established protocol that was used to prove the selectivity of GDNF for DAergic neurons (Lin et al., 1993). As shown in Fig. 7a, ³H-DA and ³H-serotonin uptake, but not ³H-GABA uptake were influenced by treatment of cultures with chromaffin granule protein. The increase in serotonin uptake was not due to a survival promoting effect of chromaffin granule protein, since the number of serotonin-positive neurons was not significantly altered (Fig. 7b).

### The trophic effect of chromaffin granule protein is accompanied by astroglial maturation and proliferation of non-DAergic cells

The molecules that have been reported to support survival of cultured DAergic neurons seem to fall in two groups. One category exerts its effects by expanding the number of astroglial cells, the other group apparently acts in a more direct fashion on DAergic neurons (Unsicker et al., 1996). To define the mechanism of the chromaffin granule protein-mediated survival of DAergic neurons cell proliferation and glial cell differentiation were studied. First, the presence of astroglial cells was analyzed at the end of the culture period by using a monoclonal antibody to GFAP. Very few, if any astroglial cells were detectable in untreated control cultures at DIV8 (Fig.8). Chromaffin granule protein-treated cultures showed a dramatic increase in GFAP-positive cells (Fig.8). In order to monitor cell proliferation, BrdU was applied to cultures, allowed to be incorporated during S-phase of the cell cycle, and detected using a monoclonal antibody against BrdU. Control cultures showed incorporation of BrdU in nuclei of very few cells, whereas cultures treated with chromaffin granule protein showed abundant labelled nuclei (Fig. 8). Double-labeling with antibodies to BrdU and TH showed that TH⁺ cells never incorporated BrdU (Fig. 8). These data indicate that the activity from chromaffin granules is mitogenic for glioblasts (possibly also for other cells present in the cultures) and induces maturation of glioblasts to become GFAP-positive astroglial cells.

### The trophic effect of chromaffin granule protein is mediated by cell proliferation and induction of astroglial cells

Treatment with the antimitotic agent 5-fluorodeoxyuridine (FDUR), and the gliotoxin aminoadipic acid (AA) at concentrations of 30 µM has previously been shown to effectively inhibit cell proliferation and astroglial cell maturation using a similar cell culture system (O'Malley et al., 1994). Antimitotic treatment was used to block proliferation and possibly abolish the survival promoting effect of chromaffin granule-derived activity. Halfmaximal concentrations of chromaffin granule protein (1:50) were used in combination with 30 µM FDUR, or AA, respectively. Treatment started at DIV1, and at DIV8 cultures were processed for TH and GFAP immunocytochemistries. As shown in Fig. 9, both treatments abolished the survival promoting affect of chromaffin granule protein without affecting basal survival, ruling out toxic side effects. These data clearly show that the survival promoting effect of chromaffin granule protein requires the numerical expansion of a cell population in this culture, most likely glioblasts which subsequently maturate to GFAP-positive astroglial cells.

### The trophic effect of chromaffin granule protein is mediated by a neurotrophic molecule ("neurotrophic factor") synthesized by a cell type present in the culture system.

Midbrain DAergic neuron cultures were set up as usually, and treatment with chromaffin granule protein (1:20) started 24h after seeding, for a period of 72 h (DIV1-DIV4). At this point, the entire culture medium was removed and replaced by serum-free medium, which was collected and renewed every 48h (DIV6 and DIV8). These media (stored at 4°C) were pooled and designated "chromaffin granule protein-conditioned medium (VP-CM)". Newly established midbrain DAergic neuron cultures were then treated with VP-CM, starting at DIV1, with two more changes of culture medium at DIV4 and DIV6, and finally processed for TH and GFAP immunocytochemistries at DIV8. As shown in Fig. 10, treating cultures with chromaffin granule protein for a period of three rather than seven days still caused a significant survival promoting effect at DIV8. More importantly, conditioned medium from cultures treated with chromaffin granule-conditioned medium (VP-CM) produced a comparable survival promoting effect on DAergic neurons. This effect was *not* accompanied by an increase in astroglial cells. Thus, chromaffin granule protein, in a first step, elicits astroglial cell maturation leading to the production and/or release of a neurotrophic factor, which, in a second step, supports DAergic neuron survival.

### Survival promoting and trophic effects are still detectable after purification of the chromaffin granule protein by chromatographical methods

Fig. 15 shows the chromatogram of the purification on Heparin Sepharose. The fractions were processed for TH and GFAP immunocytochemistry. As shown in Fig. 16, after purification on Heparin sepharose, fractions 4 and 7 caused a significant survival promoting effect. Furthermore, fraction 4 was able to induce maturation of glioblasts to become GFAP-positive astroglial cells. Proteins contained in fraction 4 were further purified using RP-HPLC. Fig. 17 shows the chromatogram of the purification. The fractions were processed for GFAP immunocytochemistry. As shown in Tab. 2, fractions 7 to 9 were able to induce maturation of glioblasts to become GFAP-positive astroglial cells.

**Tab. 1:**

| Results of the TH +-determination as shown in Fig. 16, as well as determination of GFAP+ inductive activity of fractions after purification of chromaffin granule protein on Heparin Sepharose | | |
|---|---|---|
| **Fraction No.** | **TH +** | **GFAP +** |
| Control | 100% | - |
| Fraction No. 3 | 105% | - |
| Fraction No. 4 | 165% | + |
| Fraction No. 5 | 140% | - |
| Fraction No. 6 | 110% | - |
| Fraction No. 7 | 210% | - |
| Fraction No. 8 | 150% | - |

**Tab. 2:**

| Results of the determination of GFAP+ inductive activity of fractions after purification of chromaffin granule protein using RP-HPLC | |
|---|---|
| Fraction No. | GFAP+ |
| Control | - |
| Fraction No. 7 | + |
| Fraction No. 8 | + |
| Fraction No. 9 | + |
| Fraction No. 10 | - |
| Fraction No. 11 | - |
| Fraction No. 12 | - |
| Fraction No. 13 | - |

In the Tables 1 and 2, the denotiation "+" means GFAP activity is detectable and the denotiation "-" means GFAP activity is not detectable.

### Inhibition of the EGF-receptor superfamily blocks astroglial cell maturation and, consequently, the survival promoting effect of chromaffin granule protein

FGF-2, EGF, and TGF-α have been shown to both promote DAergic neuron survival *and* to increase cell proliferation in mesencephalic cell cultures (Otto and Unsicker, 1993b; Engele and Bohn, 1991; Knüsel et al., 1990). In an initial series of experiments the chromaffin granule-derived activity is analyzed by specifically blocking its putative receptor pathway. A specific protein-tyrosine kinase inhibitor with selectivity for the EGF receptor (EGFR) signal transduction pathway is applied (Buchdunger et al. 1994). Having shown by the AA and FDUR experiments that the neurotrophic effect of the chromaffin granule protein is mediated by astrocytes (cf. Fig. 9) it was screened for suppression of the number of astroglial cells. The protocol used was as follows: 24h after seeding mesencephalic cells and again after three days, chromaffin granule protein at a concentration of 1:50 was mixed with various concentrations of the EGFR signaling blocker DAPH and applied together with fresh medium. After a total of six days cultures were fixed and processed for GFAP immunocytochemistry, As shown in Figs. 11 and 12, DAPH at 10µM concentration inhibited both the survival of DAergic neurons (Fig. 12, upper panel) and appearence of astroglial cell induced by the chromaffin granule activity. As expected, the FGF-2-mediated induction of astroglial cells could not be affected by this inhibitor (Fig. 12). When cultures were treated with TGF-α, a physiological EGFR ligand in the CNS, or EGF (10 ng/ml), only few GFAP-positive astroglial cells could be detected (Fig. 12). Their appearance could be effectively blocked by DAPH treatment (Fig. 12). These data clearly show that the chromaffin granule protein-induced appearence of astroglial cells can be specifically inhibited by DAPH, a selective inhibitor of the EGFR signaling pathway, and that the ligands TGF-α and EGF do not fully account for the effect mediated by chromaffin granule protein. The inhibitor DAPH was chosen, because it has been shown that it does not affect signal transduction of FGF-2 or PDGF induced signaling. However, the compound DAPH inhibits not only the EGFR but also p185^{c-erbB2} autophosphorylation (Buchdunger et al., 1994). To test which of the two protein kinases is involved in the chromaffin granule protein induced effects, Tyrphostin B56 which selectively inhibits the EGFR kinase autophosphorylation (Gazit et al., 1989), was used. At 10µM Tyrphostin B56 successfully inhibited the chromaffin granule protein-induced appearence of astroglial cells (Fig. 12). This finding show furtheron that the chromaffin granule-derived protein is an EGFR ligand.

### The molecule induced by chromaffin granule protein is not GDNF

In order to further define the trophic activity induced by chromaffin granule protein in mesencephalic cell culture RT-PCR was performed using RNA from mesencephalic cultures at DIV8 that had been treated with chromaffin granule protein. As shown in Fig. 13, GDNF mRNA was clearly detectable in RNA samples from the B49 gliomal cell line, from which the protein has been isolated and cloned (Lin et al., 1993). In contrast, no signal could be detected in chromaffin granule protein-treated cultures indicating that GDNF is unlikely to be the dopaminotrophic factor induced by chromaffin granule protein.

### Chromaffin granule protein protects DAergic neurons against MPP+ toxicity

MPTP- and MPP+-induced degeneration of DAergic neurons and their terminals in the nigrostriatal system resembles that seen in Parkinson's disease (Kopin and Markey, 1988). To evaluate whether the protein from chromaffin granules had the capacity to protect mesencephalic DAergic neurons from MPP+ induced death, MPP+ (1 µM) was added to mesencephalic cell cultures at DIV4, 24h subsequent to treating them with VP (1:20). Counts of surviving DAergic neurons at DIV8 indicated that treatment with VP significantly protected against MPP+ toxicity (Fig. 14).

### Summary

Protein isolated and releasable from chromaffin granules promotes the survival of mesencephalic DAergic neurons in vitro and protects them from MPP+ toxicity. The neurotrophic effect is recruited in two steps. First, chromaffin granule protein elicits cell proliferation and astroglial cell maturation involving an EGFR ligand. Second, a glia cell-derived factor distinct from GDNF promotes survival of mesencephalic DAergic neurons.

In particular, the protein constituents of the soluble content of bovine chromaffin granules trigger mechanisms leading to enhanced survival of DAergic neurons cultured from the embryonic rat midbrain floor. Thus, chromaffin cells grafted to the lesioned nigrostriatal system exert their beneficial functions through secreting biologically active molecule(s) distinct from biogenic amines. Chromaffin cells are known to synthesize, store and release neurotrophic factor(s) through the exocytotic pathway (Lachmund et al., 1994; Unsicker, 1993). Neither their number nor their molecular identity has been established as yet. FGF-2, the probably most intensely studied growth factor molecule in chromaffin cells (Stachowiak et al., 1994; Bieger et al., 1995) and potent trophic factor for developing and toxically impaired DAergic neurons in vitro and in vivo (Ferrari et al., 1989; Otto and Unsicker, 1990; 1993a, b), is not released from granules (Stachowiak et al., 1994; Bieger et al., 1995). Accordingly, FGF-2 must not be considered as a candidate for factors of the present invention. Chromaffin grafts to the brain receive *de novo* functional synaptic connections (Jousselin-Hosaja et al., 1994; Ortega et al., 1992). Moreover, grafted chromaffin cells have been shown to release their granular contents by exocytosis (Ortega et al., 1992). Exocytotic activity was reported to be most frequent in grafts to the striatum as compared to other grafting sites in the brain. It is likely therefore that catecholamines including DA and the molecule(s) causing the trophic effect would be co-released and could exert their actions near the graft site simultanously.

Concerning the mechanism of action of the chromaffin granule-derived survival promoting effect on embryonic DAergic neurons, two steps, (i) induction of cell proliferation and maturation of an increasing population of GFAP-positive astroglial cells, and (ii) stimulation of production and/or release of a dopaminotrophic factor from glial cells, can be involved. Therefore, the chromaffin granule-derived protein according to the present invention is a glial maturation and mitogenic factor. It may, however, act additionally through non-DAergic neurons. Several mitogenic growth factors, such as FGF-2, EGF, and TGF-α (Knusel et al., 1990; Casper et al., 1991; Engele and Bohn, 1991; Alexi and Hefti, 1993) have trophic effects on mesencephalic DAergic neurons which are accompanied by an increase in cell numbers, including astroglial cells. Chromaffin cells can synthesize FGF-2.

The inhibition of the EGFR superfamily blocks both the increase in astroglial cells and the survival promoting effect. DAPH, the EGFR inhibitor employed, has been shown to inhibit the EGFR protein-tyrosine kinase *in vitro* with high selectivity and also have potent *in vivo* antitumor activity (Buchdunger et al., 1994). Therefore, that the factor from chromaffin granules is a ligand for the EGFR. Since EGF and TGF-α failed to mimic the effect of the granule factor on the *in vivo* maturation and proliferation of mesencephalic astroglial progenitors, it is conceivable that another EGFR ligand is stored in chromaffin cells. There is an expanding list of EGFR ligands comprising, in addition to EGF and TGF-α, amphiregulin (Shoyab et al., 1988, 1989), heparin-binding EGF (Higashiyama et al., 1991), betacellulin (Sasada et al., 1993; Shing et al., 1993), and the most recently identified epiregulin (Toyoda et al., 1995). Another potential member is Schwannoma-derived growth factor (Kimura et al., 1990), which is homologous with amphiregulin (see Lee DY et al., 1995, for review). Furthermore, there are several mRNAs encoding related proteins belonging to the EGF superfamily, *dlk, pG2*, and *Pref-1* (Lee YL et al., 1995). Moreover, a number of viral gene products, e.g. VGF, produced by the Pox family, and proteins encoded by the Shope fibroma and myxoma viruses (see Carpenter and Wahl, 1990, for a review) are ligands for the EGFR. Transduction mechanisms of novel EGFRs interacting with still unknown ligands may also be affected by DAPH and Tyrphostin B56. However, p185c-erbB2 / HER2/neu proto-oncogene ligands, as e.g. neuregulin, glial growth factor/heregulins (Marchionni et al., 1993) can be excluded as factor candidates, since Tyrphostin B56, which selectively inhibits the EGFR kinase (IC50 5.0 µM), but not the HER1-2-kinase autophosphorylation (IC50 > 500µM; Gazit et al., 1989), fully blocked the effect of granule protein on mesencephalic glia.

Another point relevant to the present invention concerns the sorting and release mechanisms of growth factors, especially in neurons. It has widely been assumed, but not proven previously, that growth factors are released via the constitutive pathway of secretion. Evidence is emerging now that this is not exclusively true. In the PC12 pheochromocytoma cell line, which resembles chromaffin cells in many respects, transfected BDNF and NT-4 seem to be primarily released via the regulated secretory pathway (Goodman and Hefti, 1994). In contrast, hippocampal neurons secrete transfected NGF by an unconventional mechanism, which involves depolarization and potassium influx, but not external calcium (Blöchl and Thoenen, 1995). Thus, the localization of a growth factor in chromaffin granules and its regulated secretion may not constitute an unusual mode of liberation for a growth factor.

Concerning the identity of the glial derived dopaminotrophic factor induced by chromaffin granule protein according to the present invention, GDNF, the most prominent dopaminotrophic molecule, is excluded. Consistent with the results of the present invention that the dopaminotrophic effect of chromaffin granule protein involves a numerical expansion and maturation of astroglial cells is the fact that implant sites of chromaffin grafts exhibit pronounced accumulations of fibrous astroglia (Hansen et al., 1988).

### References

Alexi T, Hefti F (1993) Trophic actions of transforming growth factor α on mesencephalic dopmainergic neurons developing in culture. Neurosci 55:903-918.
Becker JB, Freed WJ (1988) Adrenal medulla grafts enhance functional activity of the striatal dopamine system following substantia nigra lesions. Brain Res 462:401-406.
Bieger SC, Henkel A, Unsicker K (1995) Localization of basic fibroblast growth factor in bovine adrenal chromaffin cells. J Neurochem 64:1521-1527.
Blöchl A, Thoenen H. (1995) Characterization of nerve growth factor (NGF) release from hippocampal neurons: Evidence for a constitutive and an unconventional sodium-dependent regulated pathway. Eur J Neurosci 7:1220-1228.
Bohn MC, Marciano F, Cupit L, Gash DM (1987) Adrenal medullary grafts promote recovery of striatal dopaminergic fibers in MPTP treated mice. Science 237:913-916.
Buchdunger E, Trinks U, Mett H, Regenass U, Müller M, Meyer T, McGlynn E, Pinna LA, Traxler P, Lydon NB (1994) 4,5-Dianilinophthalimide: A protein-tyrosine kinase inhibitor with selectivity for epidermal growth factor receptor signal transduction pathway and potent in vivo antitumor activity. Proc Natl Acad Sci USA 91: 2334.
Bottenstein J, Skaper S, Varon S, Sato G (1980) Selective survival of chick sensory ganglionic cultures utilizing serum-free supplement medium. Expl Cell Res 125:183-190.
Carpenter G, Wahl MI (1990) The epidermal growth factor family. In: Peptide growth factors and their receptors (Sporn MB, Roberts AB, eds), pp 69-171. Springer Verlag.
Casper D, Mytilineou C, Blum M (1991) EGF enhances the survival of dopamine neurons in rat embryonic mesencephalon primary cell culture. J Neurosci Res 30:372-381.
Coulter CL, McMillen IC, Browne CA (1988) The catecholamine content of the perinatal rat adrenal gland. Gen Pharmacol 19:825-828.
Ehringer H, Hornykiewicz 0 (1960) Verteilung von Noradrenalin und Dopamin (3-Hydroxytyramin) im Gehirn des Menschen und ihr Verhalten bei Erkrankungen des extrapyramidalen Systems. Klin Wschr 38:1236-1239.
Engele J, Bohn MC (1991) The neurotrophic effects of fibroblast growth factors on dopaminergic neurons in vitro are mediated by mesencephalic glia. J Neurosci 11:3070-3078.
Fann M-J, Patterson PH (1993) A novel approach to screen for cytokine effects on neuronal gene expression. J Neurochem 61:1349-1355.
Ferrari G, Minozzi M-C, Toffano G, Leon A, Skaper AD (1989) Basic fibroblast growth factor promotes the survival and development of mesencephalic neurons in culture. Devl Biol 133:140-147.
Fisher LJ, Gage FH (1993) Grafting in the mammalian central nervous system. Physiol Rev 73:583-616.
Freed WJ (1993) Neural transplantation: prospects for clinical use. Cell Transplant 2:13-31.
Gazit A, Yaissh P, Gilson C, Levittzki A (1989) Tyrphostins I: synthesis and biological activity of protein tyrosine kinase inhibitors. J Med Chem 32:2344-2352.
Goodman LJ, Hefti F (1994) Regulation and polarity of neurotrophin secretion. Soc Neurosci Abstr 20:1303.
Hansen JT, Kordower JH, Fiandaca MS, Jiao S-S, Notter MFD, Gash DM (1988) Adrenal medullary autografts into the basal ganglia of cebus monkeys: graft viability and fine structure. Exp Neurol 102:65-75
Higashiyama S, Abraham JA, Miller J, Fiddes JC, Klagsbrun M (1991) A heparin-binding growth factor secreted by macrophage-like cells that is related to EGF. Science 251 :936-939.
Jousselin-Hosaja M, Derbin C, Brisorgueil M-J, Rioux F (1994) Morphology and immunohistochemistry of the nerve endings on the chromaffin cells of adrenal medulla grafted into mouse brain. Devl Brain Res 79:321-327.
Kimura H, Fischer WH, Schubert D (1990) Structure, expression and function of a Schwannoma-derived growth factor. Nature 348:257-260.
Knusel B, Michel PP, Schwaber JS, Hefti F (1990) selective and nonselective stimulation of cholinergic and dopaminergic development in vitro by nerve growth factor, basic fibroblast growth factor, epidermal growth factor, insulin and the insulin-like growth factors I and II. J Neurosci 10:558-570.
Kopin IJ, Markey SP (1988) MPTP toxicity: implications for research in Parkinson's disease. Annu Rev Neurosci 11:81-96.
Kordower JH, Cochran E, Penn RD, Goetz CG (1991) Putative chromaffin cell survival and enhanced host-derived TH-fiber innervation following a functional adrenal medulla autograft for Parkinson's disease. Ann Neurol 29:405-412.
Krieglstein K, Unsicker K (1994) Transforming growth factor-β promotes survival of midrain dopaminergic neurons and protects them against N-methyl-4-phenylpyridinium ion toxicity. Neuroscience 63:1189-1196.
Lachmund A, Gehrke D, Krieglstein K, Unsicker K (1994) Trophic factors from chromaffin granules promote survival of peripheral and central nervous system neurons. Neuroscience 62:361-370.
Lee DC, Fenton SE, Berkowitz EA, Hissong MA (1995) Transforming growth factor σ: expression, regulation, and biological activities. Pharmacol Rev 47:51-85.
Lee YL, Helman L, Hoffman T, Laborda J (1995) *dlk, pG2* and *Pref-1* mRNAs encode similar proteins belonging to the EGF-like superfamily. Identification of polymorphic variants of this RNA. Biochim Biophys Acta 1261:223-232.
Lin LFH, Doherty DH, Lile JD, Bektesh S, Collins F (1993) GDNF - A Glial Cell Line Derived Neurotrophic Factor for Midbrain Dopaminergic Neurons. Science 260:1130-1132.
Marchionni MA, Goodearl ADJ, Chen MS, Beermingham-McDonogh O, Cassandra K, Hendricks M, Daheny F, Misumi D, Sudhalter J, Kobayashi K, Wroblewski D, Lynch C, Baldassare M, Hiles I, Davis JB, Hsuan JJ, Totty NF (1993) Glial growth factors are alternatively spliced erbB2 ligands expressed in the nervous system. Nature 362:312-318.
O'Malley EK, Sieber B-A, Morrison RS, Black IB, Dreyfus CF (1994) Nigral type I astrocytes release a soluble factor that increases dopaminergic neuron survival through mechanisms distinct from basic fibroblast growth factor. Brain Res 647:83-90.
Ortega JD, Sagen J, Pappas GD (1992) Survival and integration of bovine chromaffin cells transplanted into rat central nervous system without exogenous trophic factors. J Comp Neurol 323:13-24.
Otto D, Unsicker K (1993a) FGF-2 modulates dopamine and dopamine-related striatal transmitter systems in the intact and MPTP-lesioned mouse. Eur J Neurosci 5:927-932.
Otto D, Unsicker K (1993b) FGF-2-mediated protection of cultured mesencephalic dopaminergic neurons against MPTP and MPP+: Specificty and impact of culture conditions, non-dopaminergic neurons, and astroglial cells. J Neurosci Res 34:382-393.
Otto D, Unsicker K (1990) Basic FGF reverses chemical and morphological deficits in the nigrostriatal system of MPTP-treated mice. J Neurosci 10:1912-1921.
Sasada R, Ono Y, Taniyama Y, Shing Y, Folkman J, Igarashi K (1993) Cloning and expression of cDNA encoding human betacellulin, a new member of the EGF family. Biochem Biophys Res Commun 190:1173-1179.
Schaar DG, Sieber B-A, Dreyfus CF, Black IB (1993) Regional and cell-specific expression of GDNF in rat brain. Exp Neurol 124:368-371.
Shing Y, Christofori G, Hanahan D, Ono Y, Sasada R, Igarashi K, Folkman J (1993) Betacellulin: A mitogen from pancreatic β cell tumors. Science 259:1604-1607.
Shoyab M, McDonald VL, Bradley JG, Todaro GJ (1988) Amphiregulin: A bifunctional growth-modulating glycoprotein produced by the phorbol 12-myristate-1 3-acetate-treated human breast adenocarcinoma cell line MCF-7. Proc Natl Acad Sci USA 85:6528-6532.
Shoyab M, Plowwman GD, McDonald VL, Bradley JG, Todaro GJ (1989) Structure and function of human amphiregulin: A member of the epidermal growth factor family. Science 243:1074-1076.
Stachowiak MK, Moffett J, Joy A, Puchacz E, Florkiewicz R, Stachowiak EK (1994) Regulation of bFGF gene expression and subcellular distribution of bFGF protein in adrenal medullary cells. J Cell Biol 127:203-223.
Toyoda H, Komurasaki T, Uchida D, Takayama Y, Isobe T, Okuyama T, Hanada K (1995) Epiregulin: A novel epidermal growth factor with mitogenic activity for rat primary hepatocytes. J Biol Chem 270:7495-7500.
Unsicker K (1993) The trophic cocktail made by adrenal chromaffin cells. Exp Neurol 123:167-173.
Unsicker K, Krieglstein K (1996) Growth factors in chromaffin cells. Prog Neurobiol (in press)
Unsicker K, Stögbauer F (1992) Screening of medullary neuropeptides for putative neurotrophic effects. Int J Devl Neurosci 10:171-179.
Unsicker K, Suter-Crazzolara C, Krieglstein K (1996) Functions of growth facgtors in the development and maintenance of midbrain dopaminergic neurons: Concepts, facts, and prospects of the transforming growth factors β. In: Growth Factors as Drugs for Neurological and Sensory Disorders. (CIBA Foundation Symposium #196) pp. 70-84. Wiley, Chichester, UK.
Unsicker K, Griesser GH, Lindmar R, Löffelholz U, Wolf U (1980) Establishment, characterization and fibre outgrowth of isolated bovine adrenal medullary cells in long-term cultures. Neuroscience 5:1445-1460.
Winkler H, Apps DK, Fischer-Colbrie R (1986) The molecular function of adrenal chromaffin granules: established facts and unresolved topics. Neuroscience 18:261-290.
Winkler H, Smith AD (1975) The chromaffin granule and the storage of catecholamines. In: Handbook of Physiology (Blaschko H, Smith AD, Sayers G, eds), Section 7, Vol 6, pp321-339.

## Claims

1. Use of a chromaffin granule-derived protein factor receptor which is an epidermal grow (EGFR)- ligand, having a selective survival promoting activity in DAergic neurons, for stimulating astroglial cell maturation.

2. The use of a chromaffin granule-derived protein according to claim 1 for stimulating proliferation of non-DAergic cells.

3. Use of a chromaffin granule-derived protein as defined in claim 1 or 2 for the manufacture of a pharmaceutical composition for treating peripheral and/or CNS-disorders in man.

4. The use according to claim 3, wherein the CNS-disorder is selected from the group consisting of Parkinson's disease, Alzheimer's Disease, a neurodegenerative disorder, a peripheral neuropathy including diabetes and cisplatinium neuropathy, and a genetic or acquired peripheral nerve disease.

## Patentansprüche

1. Verwendung eines aus der Chromaffingranula stammenden Proteins, welches ein Ligand des epidermalen Wachstumsfaktorrezeptors (EGFR) ist, welches eine selektive, überlebensfördernde Aktivität in dopaminergen Neuronen aufweist, zur Stimulation der Astrozytenzellreifung.

2. Verwendung eines aus der Chromaffingranula stammenden Proteins nach Anspruch 1 zur Stimulation der Proliferation von nicht-dopaminergen Zellen.

3. Verwendung eines aus der Chromaffingranula stammenden, wie in Anspruch 1 oder 2 definierten Proteins, zur Herstellung einer pharmazeutischen Zusammensetzung zur Behandlung von peripheren und/oder ZNS-Erkrankungen im Menschen.

4. Verwendung nach Anspruch 3, wobei die ZNS-Krankheit aus der Gruppe, bestehend aus der Parkinson-Krankheit, Alzheimer-Krankheit, einer neurodegenerativen Erkrankung, einer peripheren Neuropathie, einschließlich diabetischer Neuropathie und Cisplatin-Neuropathie und einer genetischen oder erworbenen peripheren Nervenerkrankung, ausgewählt ist.

## Revendications

1. Utilisation d'une protéine dérivée de granules chromaffines, qui est un ligand de récepteur de facteur de croissance épidermique (EGFR), ayant une activité favorisant la survie sélective des neurones DAergiques, pour la stimulation de la maturation des cellules astrogliales.

2. Utilisation d'une protéine dérivée de granules chromaffines suivant la revendication 1 pour la stimulation de la prolifération de cellules non DAergiques.

3. Utilisation d'une protéine dérivée de granules chromaffines suivant la revendication 1 ou 2 pour la production d'une composition pharmaceutique destinée au traitement de troubles du système nerveux périphérique et/ou du système nerveux central chez l'homme.

4. Utilisation suivant la revendication 3, dans laquelle le trouble du système nerveux central est choisi dans le groupe consistant en la maladie de Parkinson, la maladie d'Alzheimer, un trouble neurodégénératif, une neuropathie périphérique comprenant la neuropathie diabétique et la neuropathie provoquée par le cisplatine, et une maladie génétique ou acquise des nerfs périphériques.
